# EUROPÄISCHE PATENTSCHRIFT

(11) **EP 1 832 236 B1**
(45) Veröffentlichungstag und Bekanntmachung des Hinweises auf die Patenterteilung: **19.05.2010**
(21) Anmeldenummer: 07004404.5
(22) Anmeldetag: 03.03.2007
(51) Int. Cl.: A61B 17/04, A61B 19/00

(54) **Medizinischer Knotenschieber mit einer Schneidvorrichtung zum Durchtrennen des Nahtmaterials**
Medical knot pusher with a suture cutter
Pousse-noeud à usage médical comportant un dispositif de coupure du fil de suture

(30) Priorität: 08.03.2006 DE 102006010682
(43) Veröffentlichungstag der Anmeldung: 12.09.2007
(73) Patentinhaber: Karl Storz GmbH & Co. KG, 78532 Tuttlingen (DE)
(72) Erfinder: Hahn, Martin, 88605 Boll (DE); Kreidler, Bodo, 78532 Tuttlingen (DE)
(74) Vertreter: Hofmeister, Frank

(56) Entgegenhaltungen:
- WO-A-00/69342
- WO-A-2004/069291
- WO-A-2005/084127
- WO-A2-03/059174
- DE-A1- 4 233 405
- DE-A1- 19 704 580
- US-A- 5 133 723
- US-A- 5 176 691
- US-A- 5 324 298
- US-A- 5 752 964
- US-A1- 2002 123 758

## Beschreibung

Die Erfindung betrifft einen medizinischen Knotenschieber mit einem Schaft, einer Handhabe am proximalem Ende des Schaftes, wobei der Schaft aus einem eine Führungsvorrichtung zum Halten und Führen von chirurgischem Nahtmaterial aufweisenden und in der Handhabe gelagerten Schaftrohr besteht und die am distalen Ende des Schaftes ausgebildete Führungsvorrichtung für das chirurgische Nahtmaterial als vom freien distalen Ende des Schaftrohres ausgehende Nut ausgebildet ist, die zumindest einen in proximale Richtung verlaufenden Nutabschnitt sowie einen sich daran anschließenden in tangentiale Richtung verlaufenden Nutabschnitt aufweist und wobei das Schaftrohr als Hohlrohr ausgebildet ist, in das zum Festlegen des chirurgischen Nahtmaterials in der Führungsvorrichtung ein Klemmstab einsetzbar ist.

Derartige medizinische Knotenschieber dienen dazu, einen aus einem chirurgischen Nahtmaterial vorbereiteten Knoten hin zur zu vernähenden Operationsstelle zu bewegen, um dort an der Operationsstelle nachfolgend das abschließende Verknoten vorzunehmen. Hierzu bewegt der Operateur durch distales Vorschieben des Knotenschiebers bei gleichzeitigem Festhalten der beiden Enden des Nahtmaterials den vorbereiteten Knoten hin zur zu vernähenden Operationsstelle. Häufig werden diese Knoten extrakorporal vorbereitet und über den Knotenschieber in die Körperhöhle des Patienten eingeschoben. Zum Halten und Führen des chirurgischen Nahtmaterials ist am distalen Ende des Knotenschieberschaftes eine Führungsvorrichtung ausgebildet, die ein Verlieren des Nahtmaterials beim Verschieben des Knotens verhindern soll.

Ein gattungsgemäßer Knotenschieber ist beispielsweise aus der US 5 324 298 A bekannt. Dieser bekannte Knotenschieber weist einen Schaft auf, an dessen distalem Ende eine als Nut ausgebildete Führungsvorrichtung für das Nahtmaterial ausgebildet ist, die zumindest einen in proximale Richtung verlaufenden Nutabschnitt sowie einen sich daran anschließenden in tangentiale Richtung verlaufenden Nutabschnitt aufweist. Weiterhin offenbart dieser bekannte Knotenschieber einen in den Schaft einsetzbaren Klemmstab. Nachteilig bei diesem bekannten Knotenschiebern ist, dass es immer zusätzlicher Instrumente bedarf, um beispielsweise das chirurgische Nahmaterial nach dem Plazieren des Knotens abzuschneiden.

Ein weiterer medizinischer Knotenschieber ist beispielsweise aus der DE 103 05 584 A1 bekannt. Nachteilig bei den bekannten Knotenschiebern ist, dass diese in der Regel nicht für alle Nahtmaterialien geeignet sind und es darüber hinaus auch bei diesem Instrument immer zusätzlicher Instrumente bedarf, um beispielsweise das chirurgische Nahmaterial nach dem Plazieren des Knotens abzuschneiden.

Weitere medizinische Knotenschieber sind aus der WO 00/69342 A2 sowie der US 5 752 964 A bekannt.

Der aus der WO 00/69342 A2 bekannte medizinische Knotenschieber weist ein koaxial auf dem rohrförmigen Schaft gelagertes und in Längsrichtung des Schaftes verschiebbares Außenrohr auf, dessen distales Ende eine geschärfte Kante zum Durchtrennen des Nahtmaterials aufweist. Die Fadenführungsvorrichtung dieses bekannten Knotenschiebers wird durch eine am distalen Ende des Schaftes angeordnete und in den Schaft einziehbare offene Drahtschlinge gebildet. Die Ausbildung der Schneidkante als das Schaftrohr koaxial umgebende starre Schneidkante weist den Nachteil auf, dass das abzutrennende Nahtmaterial nicht von der Schneidkante ergriffen wird, sondern zwischen dem Schaftrohr und dem axialverschiebbar auf dem Schaftrohr gelagerten Schneidrohr eingeklemmt wird.

Aus der US 5 752 964 A ist ein Knotenschieber mit einem innerhalb des hohlen Schaftes gelagerten Betätigungsstab bekannt, an dessen distalem Ende eine als Federzunge ausgebildete Schneidklinge zum Abtrennen des Nahtmaterials angeordnet ist. Nachteilig an dieser bekannten Konstruktion mit einer innerhalb des Schaftes angeordneten Schneidvorrichtung ist, dass in den hohlen Schaft kein Klemmstab mehr einsetzbar ist, um das Nahtmaterials in der Führungsvorrichtung festlegen zu können.

Davon ausgehend liegt der Erfindung die **Aufgabe** zugrunde, einen medizinischen Knotenschieber der eingangs genannten Art so auszugestalten, dass dieser bei einfacher Handhabung vielseitig verwendbar ist.

Die **Lösung** dieser Aufgabenstellung ist erfindungsgemäß **dadurch gekennzeichnet, dass** am Schaft zusätzliche eine Schneidvorrichtung zum Durchtrennen des in der Führungsvorrichtung angeordneten chirurgischen Nahtmaterials angeordnet ist, wobei die Schneidvorrichtung aus einem auf das Schaftrohr aufsetzbaren und im Wesentlichen in Axialrichtung des Schaftrohres verlagerbaren Schneidrohr besteht, an dessen distalem Ende mindestens eine Schneidkante angeordnet ist, die am distalen Ende einer im Schneidrohr freigeschnittenen Federzunge ausgebildet ist, wobei die Federzunge radial nach innen in Richtung auf das Schaftrohr vorgespannt ist.

Die erfindungsgemäße Ausbildung der Schneidvorrichtung als auf das Schaftrohr aufsetzbares Schneidrohr ist insbesondere aus räumlichen Gründen vorteilhaft, da auch die Führungsvorrichtung für das chirurgische Nahtmaterial am Schaft angeordnet ist. Die Anordnung des Schneidrohres auf dem das Nahtmaterial in der Führungsvorrichtung haltenden Schaftrohr ermöglicht bei einfacher Konstruktion ein zuverlässiges Abtrennen des Nahtmaterials. Hierdurch wird die Handhabung für den Operateur deutlich vereinfacht, da kein zusätzliches Instrument ins Operationsgebiet gebracht werden muss, welches insbesondere bei endoskopischen Operationen räumlich sehr beengt ist.

Das Abtrennen des chirurgischen Nahtmaterials mittels der Schneidvorrichtung erfolgt über mindestens eine am distalen Ende des Schneidrohres angeordnete Schneidkante.

Zur Verbesserung der Schneidwirkung ist die Schneidkante am distalen Ende einer freigeschnittenen Federzunge ausgebildet, die radial nach innen in Richtung auf das Schaftrohr vorgespannt ist.

Das Durchtrennen des Nahtmaterials mittels der Schneidvorrichtung kann erfindungsgemäß dadurch verbessert werden, dass die mindestens eine Schneidkante zum Durchtrennen des chirurgischen Nahtmaterials mit mindestens einem Nutabschnitt der Führungsvorrichtung zusammenwirkt.

Das Schneidrohr ist vorteilhafterweise über einen an der Handhabe angeordneten Betätigungsmechanismus axialverschiebbar auf dem Schaftrohr gelagert.

Zur Ausbildung des Betätigungsmechanismus wird mit der Erfindung vorgeschlagen, dass der Betätigungsmechanismus als koaxial auf dem Schaftrohr angeordnete Hülse ausgebildet ist, die ein Kopplungselement zum Festlegen des Schneidrohres sowie einen Schieber zum Bewegen des Schneidrohres aufweist. Vorteilhafterweise ist der Schieber in zumindest einer Endposition arretierbar.

Gemäß einer bevorzugten Ausführungsform der Erfindung schließt sich an den in tangentiale Richtung verlaufenden Nutabschnitt ein zweiter, sich wiederum in axialer Richtung erstreckender Nutabschnitt an, wodurch das Führen und Halten des Nahtmaterials in der Führungsvorrichtung weiter verbessert werden kann.

Schließlich wird mit der Erfindung vorgeschlagen, dass im distalen Ende des Klemmstabes eine in Längsrichtung des Klemmstabes verlaufende Nut ausgebildet ist, die im in das Schaftrohr eingesetzten Zustand mit dem zweiten in axialer Richtung verlaufenden Nutabschnitt der Führungsvorrichtung fluchtet.

Weitere Merkmale und Vorteile der Erfindung ergeben sich anhand der zugehörigen Zeichnung, in der ein Ausführungsbeispiel eines erfindungsgemäßen medizinischen Knotenschiebers nur beispielhaft dargestellt ist. In der Zeichnung zeigt:
- Fig. 1: eine Seitenansicht eines erfindungsgemäßen medizinischen Knotenschiebers;
- Fig. 2: eine Draufsicht auf den Knotenschieber gemäß Fig. 1;
- Fig. 3: einen Schnitt entlang der Linie III-III gemäß Fig. 2, jedoch ohne Schneidrohr und Klemmstab;
- Fig. 4: eine vergrößerte Darstellung des Details IV gemäß Fig. 1, jedoch ohne Nahtmaterial;
- Fig. 5: eine vergrößerte Darstellung des Details V gemäß Fig. 2, jedoch ohne Nahtmaterial und
- Fig. 6: eine vergrößerte Schnittdarstellung des Details VI gemäß Fig. 1.

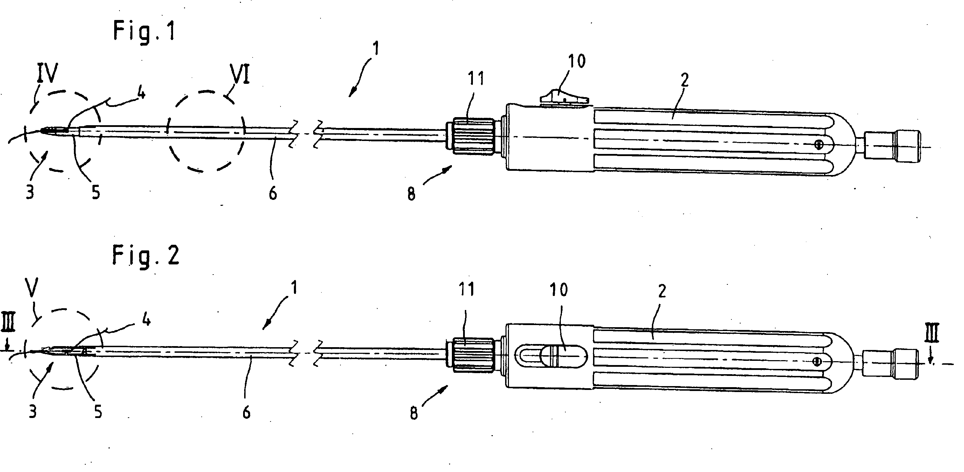

Der in den Abbildungen Fig. 1 bis Fig. 3 dargestellte medizinische Knotenschieber besteht im Wesentlichen aus einem sich längs erstreckenden Schaft 1, der proximalseitig in einer Handhabe 2 gelagert ist und an dessen distalem Ende eine Führungsvorrichtung 3 zum Halten und Führen von chirurgischem Nahtmaterial 4 ausgebildet ist.

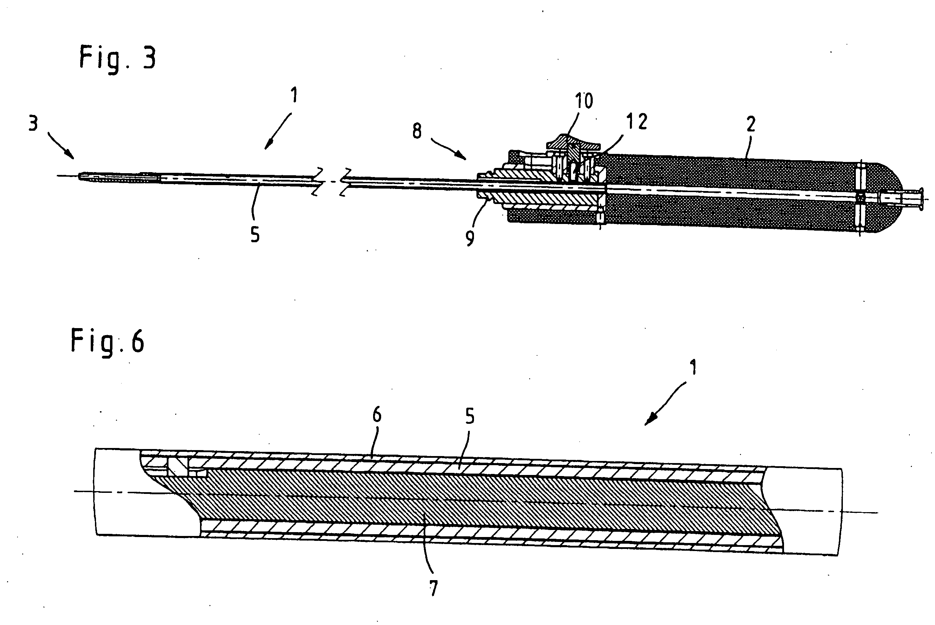

Wie insbesondere aus der vergrößerten Detailansicht gemäß Fig. 6 ersichtlich, besteht der Schaft 1 bei der dargestellten Ausführungsform aus einem in der Handhabe 2 gelagerten hohlen Schaftrohr 5, einem auf das Schaftrohr 5 aufsetzbaren und dieses koaxial umgebenden Schneidrohr 6 sowie einem vom proximalen Ende her in das hohle Schaftrohr 5 einsetzbaren Klemmstab 7, wobei das Schneidrohr 6 über einen Betätigungsmechanismus 8 axialverschiebbar auf dem Schaftrohr 5 gelagert ist.

Der Betätigungsmechanismus 8 ist dabei, wie aus Fig. 3 ersichtlich, als koaxial auf dem Schaftrohr 5 angeordnete Hülse ausgebildet, die distalseitig ein Kopplungselement 9 zum Festlegen des Schneidrohres 6 sowie einen Schieber 10 aufweist, über den der Betätigungsmechanismus 8 in Längsrichtung des Schaftrohres 5 hin und her schiebbar ist. Das Kopplungselement 9 zum Festlegen des Schneidrohres 6 ist bei der dargestellten Ausführungsform als Schraubverbindung so ausgebildet, dass das Schneidrohr 6 mittels einer Überwurfmutter 11 am Betätigungsmechanismus 8 festlegbar ist. Um ein versehentliches Verlagern des Betätigungsmechanismus 8 und somit auch des Schneidrohres 6 zu verhindern, ist der Schieber 10 des Betätigungsmechanismus 8 zumindest in der zum proximalen Ende der Handhabe 2 zurückgezogenen Grundstellung, wie diese in Fig. 1 und 2 dargestellt ist, arretierbar. Das Arretieren des Schiebers 10 erfolgt bei dieser Ausführungsform über ein federbelastetes Sperrelement 12 derart, dass der Schieber 10 erst eingedrückt werden muss, bevor ein Verschieben des Betätigungsmechanismus 8 erfolgen kann.

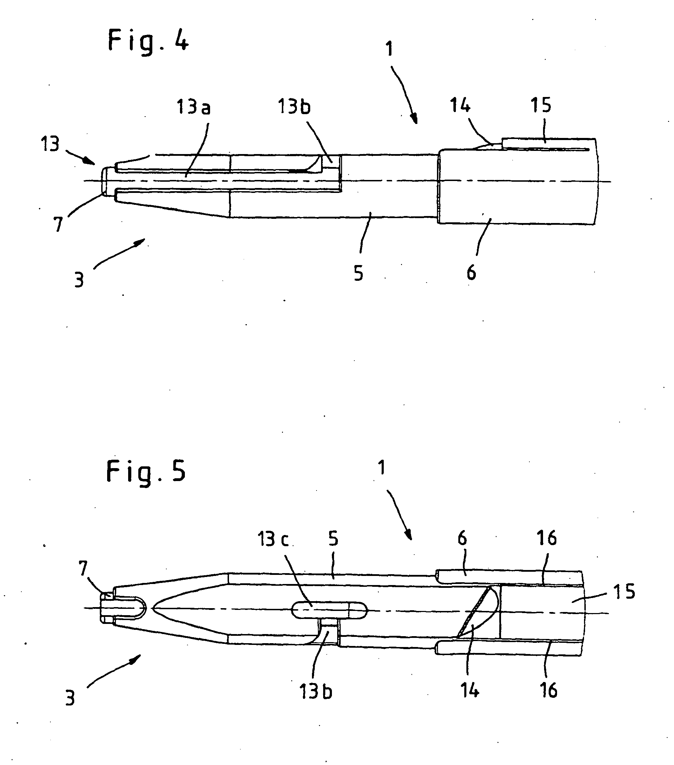

Der Aufbau der Führungsvorrichtung 3 zum Halten und Führen des chirurgischen Nahtmaterials 4 ist insbesondere den vergrößerten Detailansichten gemäß Fig. 4 und Fig. 5 zu entnehmen. Die Führungsvorrichtung 3 ist bei dieser Ausgestaltungsform als sich vom freien distalen Ende des Schaftrohres 5 ausgehende Nut 13 ausgebildet ist, die einen ersten in proximale Richtung verlaufenden Nutabschnitt 13a sowie einen sich daran anschließenden in tangentiale Richtung verlaufenden Nutabschnitt 13b aufweist, an den sich ein zweiter, wiederum in axialer Richtung sich nach proximal und distal erstreckender Nutabschnitt 13c anschließt.

Um das in der Nut 13 der Führungsvorrichtung 3 angeordnete chirurgische Nahtmaterial 4 in der Führungsvorrichtung 3 zu halten, das heißt ein Herausrutschen des Nahtmaterials 4 aus der Nut 13 zu verhindern, ist in das hohle Schaftrohr 5 vom proximalen Ende her der Klemmstab 7 einschiebbar, der bis zum distalen Ende des Schaftrohres 5 reicht und so das in der Führungsvorrichtung 3 angeordnete Nahtmaterial 4 klemmend in der Nut 13 hält. Damit das Nahtmaterial 4 durch den Klemmstab 7 aber einerseits nicht beschädigt wird und andererseits noch eine gewisse Verlagerung des Nahtmaterials in Längsrichtung des Nutabschnitts 13c möglich ist, ist im distalen Ende des Klemmstabes 7 eine in Längsrichtung des Klemmstabes 7 verlaufende Nut ausgebildet ist, die im in das Schaftrohr 5 eingesetzten Zustand mit dem zweiten in axialer Richtung verlaufenden Nutabschnitt 13c der Führungsvorrichtung 3 fluchtet. Der Klemmstab 7 verhindert somit in erster Linie, dass das Nahtmaterial 4 über die Nutabschnitte 13b und 13a wieder aus der Führungsvorrichtung herausgleiten kann. Das lagegerechte Ausrichten der im Klemmstab 7 ausgebildeten Nut zu Nutabschnitt 13c kann dadurch erleichtert werden, dass einerseits am Klemmstab 7 und andererseits in der Bohrung des hohlen Schaftrohres 5 miteinander korrespondierende Führungen ausgebildet sind, die ein exaktes Einsetzen des Klemmstabes 7 in das Schaftrohr 5 ermöglichen.

Wie weiterhin aus Fig. 4 ersichtlich, ist bei der dargestellten Ausführungsform des Schneidrohres 6 am distalen Ende des Schneidrohres 6 eine Schneidkante 14 angeordnet, die zum scherenden Durchtrennen des im Nutabschnitt 13c der Führungsvorrichtung 3 gelagerten Nahtmaterials 4 dient. Die Schneidwirkung der Schneidkante 14 wird bei der dargestellten Ausführungsform dadurch verbessert, dass die Schneidkante 14 am distalen Ende einer Federzunge 15 ausgebildet ist, die durch zwei parallele, sich nach proximal erstreckende Einschnitte 16 im Schneidrohr 6 biegeelastisch freigeschnitten ist. Um ein glattes Durchtrennen des Nahtmaterials 4 zu gewährleisten, ist die Federzunge 15 radial nach innen in Richtung auf das Schaftrohr 5 so vorgespannt, dass die Schneidkante 14 beim verschieben des Schneidrohres 6 über die äußere Mantelfläche des Schaftrohres 5 gleitet, wodurch ein Verklemmen oder Verquetschen des Nahtmaterials 4 verhindert wird.

Die Montage und Handhabung des zuvor beschriebenen medizinischen Knotenschiebers geschieht wie folgt:

Ausgehend von der in Fig. 3 geschnitten dargestellten Grundform des Knotenschiebers wird zunächst vom distalen Ende her das Schneidrohr 6 auf das hohle Schaftrohr 5 aufgeschoben und mittels der Überwurfmutter 11 am Kopplungselement 9 des Betätigungsmechanismus 8 festgelegt, so dass das Schneidrohr 6 durch Verschieben des Betätigungsmechanismus 8 in Axialrichtung des Schaftrohres 5 hin und her schiebbar auf dem Schaftrohr 5 gelagert ist.

Nachfolgend fädelt der Operateur ein Ende des mit einem vorbereiteten Knoten versehenen chirurgischen Nahtmaterials 4 so in der Führungsvorrichtung 3 des Schaftrohres 5 ein, dass das Nahtmaterial 4 über die Nutabschnitte 13a und 13b im Nutabschnitt 13c zu liegen kommt. Damit das Nahtmaterial 4 nicht wieder über die Nutabschnitte 13b und 13a aus der Führungsvorrichtung 3 herausrutschen kann, wird nun vom proximalen Ende des Schaftrohres 5 her der Klemmstab 7 in das Schaftrohr 5 eingeschoben, bis dieser das Nahtmaterial 4 im Nutabschnitt 13c klemmend festlegt.

Das eigentliche Verschieben des in der Regel extrakorporal vorbereiteten Knotens hin zur zu vernähenden Operationsstelle erfolgt durch distales Vorschieben des Knotenschiebers bei gleichzeitigem Festhalten der proximalen Enden des Nahtmaterials 4, beispielsweise mit einer Fasszange. Da der Klemmstab 7 das Nahtmaterial 4 im Nutabschnitt 13c nur so klemmt, dass dieses nicht seitlich über den Nutabschnitt 13b aus der Führungsvorrichtung 3 heraustreten kann, ist trotz Klemmung des Nahtmaterials 4 über den Klemmstab 7 eine axiale Verlagerung des Nahtmaterials 4 in der Führungsvorrichtung 3 und somit ein Verschieben des Knotens entlang des Nahtmaterials 4 möglich.

Sobald der Knoten an der zu vernähenden Stelle des Operationsgebiets angelangt ist, kann der Operateur das in der Führungsvorrichtung 3 gelagerte Nahtmaterial 4 durch Betätigen des Schneidrohres 6 durchtrennen. Hierzu drückt der Operateur den Schieber 10 des Betätigungsmechanismus 8 entgegen der Kraft des federbelasteten Sperrelements 12 nach unten und schiebt den Schieber 10 und somit auch das über das Kopplungselement 9 kraftschlüssig mit dem Betätigungsmechanismus 8 verbundene Schneidrohr 6 in distale Richtung des Schaftrohres 5 nach vorne, bis die Schneidkante 14 des Schneidrohres 6 das im Nutabschnitt 13c der Führungsvorrichtung 3 klemmend gelagerte Nahtmaterial 4 durchtrennt.

Anschließend wird der Knotenschieber wieder nach proximal aus dem Operationsgebiet herausgezogen, wobei das die Naht bildende abgetrennte Nahtmaterial 4 distalseitig aus der Führungsvorrichtung 3 heraustritt.

Durch die Integration der Schneidvorrichtung in den Knotenschieber kann auf die ansonsten aus der Praxis bekannte Verwendung einer zusätzlichen Schere zum Durchtrennen des Nahtmaterials 4 verzichtet werden, wodurch die Arbeit des Operateurs deutlich erleichtert wird.

### Bezugszeichenliste

- 1: Schaft
- 2: Handhabe
- 3: Führungsvorrichtung
- 4: Nahtmaterial
- 5: Schaftrohr
- 6: Schneidrohr
- 7: Klemmstab
- 8: Betätigungsmechanismus
- 9: Kopplungselement
- 10: Schieber
- 11: Überwurfmutter
- 12: Sperrelement
- 13: Nut
- 13a: erster axialer Nutabschnitt
- 13b: radialer Nutabschnitt
- 13c: zweiter axialer Nutabschnitt
- 14: Schneidkante
- 15: Federzunge
- 16: Einschnitt

## Patentansprüche

1. Medizinischer Knotenschieber mit einem Schaft (1), einer Handhabe (2) am proximalem Ende des Schaftes (1), wobei der Schaft (1) aus einem eine Führungsvorrichtung (3) zum Halten und Führen von chirurgischem Nahtmaterial (4) aufweisenden und in der Handhabe (2) gelagerten Schaftrohr (5) besteht und die am distalen Ende des Schaftes (1) ausgebildete Führungsvorrichtung (3) für das chirurgische Nahtmaterial (4) als vom freien distalen Ende des Schaftrohres (5) ausgehende Nut (13) ausgebildet ist, die zumindest einen in proximale Richtung verlaufenden Nutabschnitt (13a) sowie einen sich daran anschließenden in tangentiale Richtung verlaufenden Nutabschnitt (13b) aufweist und wobei das Schaftrohr (5) als Hohlrohr ausgebildet ist, in das zum Festlegen des chirurgischen Nahtmaterials (4) in der Führungsvorrichtung (3) ein Klemmstab (7) einsetzbar ist,
**dadurch gekennzeichnet,**
**dass** am Schaft (1) zusätzlich eine Schneidvorrichtung zum Durchtrennen des in der Führungsvorrichtung (3) angeordneten chirurgischen Nahtmaterials (4) angeordnet ist, wobei die Schneidvorrichtung aus einem auf das Schaftrohr (5) aufsetzbaren und im Wesentlichen in Axialrichtung des Schaftrohres (5) verlagerbaren Schneidrohr (6) besteht, an dessen distalem Ende mindestens eine Schneidkante (14) angeordnet ist, die am distalen Ende einer im Schneidrohr (6) freigeschnittenen Federzunge (15) ausgebildet ist, wobei die Federzunge (15) radial nach innen in Richtung auf das Schaftrohr (5) vorgespannt ist.

2. Medizinischer Knotenschieber nach Anspruch 1, **dadurch gekennzeichnet, dass** die mindestens eine Schneidkante (14) zum Durchtrennen des chirurgischen Nahtmaterials (4) mit mindestens einem Nutabschnitt (13a, 13b, 13c) der Führungsvorrichtung (3) zusammenwirkt.

3. Medizinischer Knotenschieber nach Anspruch 1 oder 2, **dadurch gekennzeichnet, dass** das Schneidrohr (6) über einen an der Handhabe (2) angeordneten Betätigungsmechanismus (8) axialverschiebbar auf dem Schaftrohr (5) gelagert ist.

4. Medizinischer Knotenschieber nach Anspruch 3, **dadurch gekennzeichnet, dass** der Betätigungsmechanismus (8) als koaxial auf dem Schaftrohr (5) angeordnete Hülse ausgebildet ist, die ein Kopplungselement (9) zum Festlegen des Schneidrohres (6) sowie einen Schieber (10) zum Bewegen des Schneidrohres (6) aufweist.

5. Medizinischer Knotenschieber nach Anspruch 4, **dadurch gekennzeichnet, dass** der Schieber (10) zumindest in einer Endposition arretierbar ist.

6. Medizinischer Knotenschieber nach einem der Ansprüche 1 bis 5, **dadurch gekennzeichnet, dass** sich an den in tangentiale Richtung verlaufenden Nutabschnitt (13b) ein zweiter, sich wiederum in axialer Richtung erstreckender Nutabschnitt (13c) anschließt.

7. Medizinischer Knotenschieber nach einem der Ansprüche 1 bis 6, **dadurch gekennzeichnet, dass** im distalen Ende des Klemmstabes (7) eine in Längsrichtung des Klemmstabes (7) verlaufende Nut ausgebildet ist, die im in das Schaftrohr (5) eingesetzten Zustand mit dem zweiten in axialer Richtung verlaufenden Nutabschnitt (13c) der Führungsvorrichtung (3) fluchtet.

## Claims

1. Medical knot pusher with a shaft (1), a handle (2) at the proximal end of the shaft (1), the shaft (1) comprising a shaft tube (5) having a guide device (3) for holding and guiding surgical suture material (4) and being mounted in the handle (2), and the guide device (3) for the surgical suture material (4) formed at the distal end of the shaft (1) being designed as a groove (13) originating at the free distal end of the shaft tube (5), which groove (13) has at least one groove section (13a) running in the proximal direction and, connected thereto, a groove section (13b) running in the tangential direction, and the shaft tube (5) being designed as a hollow tube into which a clamping rod (7) can be inserted for fixing the surgical suture material (4) in the guide device (3),
**characterized**
**in that** a cutting device for severing the surgical suture material (4) arranged in the guide device (3) is additionally arranged on the shaft (1), with the cutting device comprising a cutting tube (6) which can be placed onto the shaft tube (5) and can be displaced substantially in the axial direction of the shaft tube (5), with at least one cutting edge (14) being arranged at the distal end of said cutting tube, which cutting edge is formed on the distal end of a spring tongue (15) cut free in the cutting tube (6), with the spring tongue (15) being prestressed radially inwards in the direction of the shaft tube (5).

2. Medical knot pusher according to Claim 1,
**characterized in that** the at least one cutting edge (14) interacts with at least one groove section (13a, 13b, 13c) of the guide device (3) for severing the surgical suture material (4).

3. Medical knot pusher according to Claim 1 or 2, **characterized in that** the cutting tube (6) is mounted in an axially displaceable fashion on the shaft tube (5) via an actuation mechanism (8) arranged on the handle (2).

4. Medical knot pusher according to Claim 3,
**characterized in that** the actuation mechanism (8) is designed as a sleeve arranged coaxially on the shaft tube (5), which sleeve has a coupling element (9) for fixing the cutting tube (6) and a slide (10) for moving the cutting tube (6).

5. Medical knot pusher according to Claim 4,
**characterized in that** the slide (10) can be locked in at least one end position.

6. Medical knot pusher according to one of Claims 1 to 5, **characterized in that** a second groove section (13c) which in turn extends in the axial direction adjoins the groove section (13b) running in the tangential direction.

7. Medical knot pusher according to one of Claims 1 to 6, **characterized in that** a groove running in the longitudinal direction of the clamping rod (7) is formed in the distal end of the clamping rod (7), which groove, in the state where it is inserted into the shaft tube (5), is flush with the second groove section (13c) of the guide device (3) running in the axial direction.

## Revendications

1. Pousse-noeud médical comportant une tige (1), une poignée (2) à l'extrémité proximale de la tige (1), la tige (1) étant constituée d'un tube de tige (5) logé dans la poignée (2) et présentant un dispositif de guidage (3) destiné à maintenir et guider du matériel de suture chirurgical (4) et le dispositif de guidage (3) formé à l'extrémité distale de la tige (1) pour le matériel de suture chirurgical (4) étant conçu sous forme d'une gorge (13) partant de l'extrémité distale libre du tube de tige (5) et présentant au moins un tronçon de gorge (13a) dans la direction proximale ainsi qu'un tronçon de gorge (13b) adjacent dans la direction tangentielle et le tube de tige (5) étant conçu sous forme de tube creux dans lequel peut être insérée une baguette de serrage (7) pour immobiliser le matériel de suture chirurgical (4) dans le dispositif de guidage (3),
**caractérisé en ce qu'**est disposé en outre sur la tige (1) un dispositif de coupe pour trancher le matériel de suture chirurgical (4) disposé dans le dispositif de guidage (3), le dispositif de coupe étant composé d'un tube de coupe (6) pouvant être enfilé sur le tube de tige (5) et déplacé sensiblement dans la direction axiale du tube de tige (5) et à l'extrémité distale duquel est disposée au moins une arête de coupe (14) formée à l'extrémité distale d'une languette ressort (15) libérée par découpage dans le tube de coupe (6), la languette ressort (15) étant précontrainte radialement vers l'intérieur dans la direction du tube de tige (5).

2. Pousse-noeud médical selon la revendication 1, **caractérisé en ce que** la au moins une arête de coupe (14) destinée à trancher le matériel de suture chirurgical (4) coopère avec au moins un tronçon de gorge (13a, 13b, 13c) du dispositif de guidage (3).

3. Pousse-noeud médical selon la revendication 1 ou 2, **caractérisé en ce que** le tube de coupe (6) est logé de façon à pouvoir être déplacé axialement sur le tube de tige (5) par l'intermédiaire d'un mécanisme d'actionnement (8) disposé sur la poignée (2).

4. Pousse-noeud médical selon la revendication 3, **caractérisé en ce que** le mécanisme d'actionnement (8) est conçu sous forme de douille disposée coaxialement sur le tube de tige (5) et qui présente un élément de couplage (9) pour immobiliser le tube de coupe (6) ainsi qu'un poussoir (10) pour déplacer le tube de coupe (6).

5. Pousse-noeud médical selon la revendication 4, **caractérisé en ce que** le poussoir (10) peut être arrêté au moins en une position finale.

6. Pousse-noeud médical selon une des revendications 1 à 5, **caractérisé en ce qu'**un deuxième tronçon de gorge (13c) s'étendant à nouveau dans la direction axiale fait suite au tronçon de gorge (13b) dans la direction tangentielle.

7. Pousse-noeud médical selon une des revendications 1 à 6, **caractérisé en ce qu'**à l'extrémité distale de la baguette de serrage (7) est formée une gorge dans la direction longitudinale de la baguette de serrage (7) et qui, à l'état introduite dans le tube de tige (5), est alignée avec le deuxième tronçon de gorge (13c), dans la direction tangentielle, du dispositif de guidage (3).
